# EUROPÄISCHE PATENTSCHRIFT

(11) **EP 0 935 751 B1**
(45) Veröffentlichungstag und Bekanntmachung des Hinweises auf die Patenterteilung: **09.01.2002**
(21) Anmeldenummer: 97913178.6
(22) Anmeldetag: 25.10.1997
(51) Int. Cl.: G01N 33/30, G01N 19/02

(54) **VERFAHREN ZUR ERMITTLUNG DER FUNKTIONSTAUGLICHKEIT VON SCHMIERÖLEN**
PROCESS FOR DETERMINING THE LUBRICANT POWER OF LUBRICANT OILS
PROCEDE POUR DETERMINER LE POUVOIR LUBRIFIANT D'HUILES LUBRIFIANTES

(30) Priorität: 31.10.1996 DE 19644029
(43) Veröffentlichungstag der Anmeldung: 18.08.1999
(73) Patentinhaber: ZF FRIEDRICHSHAFEN Aktiengesellschaft, 88038 Friedrichshafen (DE)
(72) Erfinder: THELEN, Edgar, D-88048 Friedrichshafen (DE)
(86) Internationale Anmeldenummer: EP9705904
(87) Internationale Veröffentlichungsnummer: WO9819157

(56) Entgegenhaltungen:
- FR-A- 2 277 342
- US-A- 2 909 056
- US-A- 3 045 471
- US-A- 3 913 377

## Beschreibung

Die Erfindung betrifft ein Verfahren zur Ermittlung der Funktionstauglichkeit von Schmierölen mit den Merkmalen nach dem Oberbegriff von Anspruch 1, wie es etwa aus US 39 13 377 bekannt ist.

Der Einsatz von Schmierölen, insbesondere von Schmierölen, sogenannten "Automatic Transmission Fluids" (ATF) in einer schlupfgeregelten Kupplung setzt genaue Kenntnisse des Reibwertverlaufs als Funktion der Gleitgeschwindigkeit voraus. Entspricht der Verlauf des Reibwerts über der Gleitgeschwindigkeit im Betriebsbereich nicht einem progressiven Verlauf, ist damit zu rechnen, daß im Fahrzeug selbsterregte Reibschwingungen auftreten. Damit ist eine ordnungsgemäße Funktion schlupfgeregelter Kupplungen, z. B. bei geregelten Wandlerüberbrückungskupplungen nicht mehr gegeben.

In der Regel werden Schmieröle hinsichtlich ihrer Tauglichkeit für den Einsatz in schlupfgeregelten Kupplungen mit aufwendigen Prüfstandsaufbauten mit originalen Einbauteilen bewertet. Allerdings sind die gewonnenen Werte nicht ohne weiteres auf die Funktionstauglichkeit nach bestimmten Gebrauchsdauern der Schmieröle zu übertragen. Weitere Untersuchungen in regelmäßigen Abständen sind erforderlich, aber mit vertretbarem Kosten- und Zeitaufwand nicht möglich. Aus den gleichen Gründen scheidet meistens eine breitgefächerte Erprobung in Fahrzeug- und/oder Komponententests aus. Die Kenntnis dieser Werte ist jedoch unerläßlich, wenn man für Schmieröle eine langfristige Funktionsfähigkeit, ja sogar eine Lebensdauerfunktionsfähigkeit garantieren muß.

Der Erfindung liegt die Aufgabe zugrunde, ein Verfahren und eine Vorrichtung zu schaffen, mit der die Funktionstauglichkeit von Schmierölen für schlupfgeregelte Kupplungen mit geringem Aufwand ermittelt werden kann, so daß auch Aussagen über eine langfristige Funktionstauglichkeit möglich sind.

Sie wird gemäß der Erfindung durch die Merkmale des Anspruchs 1 gelöst.

Für das erfindungsgemäße Verfahren und die Vorrichtung werden keine originalen Einbauteile benötigt. Ferner sind nur kleine Ölmengen, ca. 100 ml, erforderlich. Dadurch ergibt sich eine Kosten-- und Zeitersparnis sowohl in bezug auf den Prüfstandsaufbau als auch hinsichtlich der Versuchsdurchführung. Somit kann nunmehr die Funktionstauglichkeit in Begleitung von Fahrzeug- oder Prüfstandsversuchen erfaßt werden, weil nur ein kleiner Bruchteil des Schmieröls dem Schmierölkreislauf zu Prüfzwecken entnommen wird und das restliche Schmieröl im Langzeitversuch verbleibt.

Zweckmäßigerweise wird die Prüfölmenge auf unter 200 ml begrenzt. Hierzu kann der Prüfbehälter und die darin enthaltenen Teile, z. B. der Probenhalter usw. entsprechend dimensioniert werden, damit auch bei geringer Prüfölmenge die Kontaktflächen während der Prüfung immer ausreichend mit Prüföl versorgt sind. Ferner können zusätzliche Verdrängerkörper im Prüfbehälter angeordnet werden, um die gewünschte Prüfölmenge zu erhalten. Für die Entwicklung von geeigneten Schmierölen ist von entscheidender Bedeutung, die Änderung der Reibwertcharakteristik als Funktion der Gebrauchsdauer zu kennen. Mit dem erfindungsgemäßen Verfahren und der entsprechenden Vorrichtung ist es möglich, Reibungssysteme, wie sie in schlupfgeregelten Kupplungen vorliegen, bezüglich ihrer Reibwertcharakteristik in verschiedenen Behandlungs- und Gebrauchszuständen zu beschreiben. Das Verfahren und die Vorrichtung lassen sich somit bei Schmiermitteln zur Kontrolle von Herstellchargen, bei der Schmierölentwicklung, begleitend bei Prüfstands- und Feldversuchen, nach labormäßigen Alterungsprozessen und zur Bewertung neuer Reibbeläge einsetzen.

In der Zeichnung ist ein Ausführungsbeispiel der Erfindung dargestellt.
Es zeigt:
- Fig. 1: in schematischer Weise einen Aufbau einer erfindungsgemäßen Vorrichtung;
- Fig. 2: einen Bereich II in Fig. 1 in vergrößerter Ansicht und
- Fig. 3: den Verlauf von Reibwertcharakteristiken.

Ein Motor 1 treibt über eine Drehmomentmeßnabe 2, eine Antriebswelle 4 und eine Ausgleichsverbindung 3 eine Stützscheibe 5 an, an der mit einer Schraube 22 eine Ringscheibe 6 befestigt ist. Die Ringscheibe 6 bildet einen Reibpartner und besitzt eine Ringfläche 7, mit der sie an einem anderen Reibpartner anliegt. Dieser wird von einem Reibbelag 8 gebildet, der auf einen Grundkörper 9 aufgebracht ist. Der Grundkörper 9 ist mittels einer Schraube 21 mit einem Probenhalter 10 verschraubt, der in einem Prüfbehälter 14 über eine Momentenabstützung 16 drehfest gehalten ist und über ein Spitzenlager 11 nach allen Seiten hin kippbeweglich gelagert ist. Anstelle des Spitzenlagers 11, das von einem doppelkegelförmigen Körper gebildet wird, kann auch eine Kugel verwendet werden, die in entsprechende Kalotten der benachbarten Teile eingreift.

Der Prüfbehälter 14 ist doppelwandig und kann temperiert werden, so daß Schmieröle und Reibpaarungen bei verschiedenen Betriebstemperaturen untersucht werden können. Er ist bis zu einem mit 13 bezeichneten Ölspiegel mit Prüföl gefüllt.

Der Prüfbehälter 14 ruht auf einem Prüftisch 15. Ein Lastaufgabesystem 20 drückt den Prüftisch 15 mit dem Probenhalter 10, dem Grundkörper 9 und dem Reibbelag 8 über einen Hebelmechanismus 18, der sich an einem Fundament 19 gelenkig abstützt, gegen die Ringfläche 7 der Ringscheibe 6.

Das erfindungsgemäße Verfahren wird wie folgt durchgeführt:

Die beiden Reibpartner, die von der Ringscheibe 6 einerseits und dem Grundkörper 9 mit dem Reibbelag 8 andererseits gebildet werden, werden aufgespannt und der Prüfbehälter 14 mit dem zu untersuchenden Prüföl gefüllt. Die beiden Reibpartner entsprechen im wesentlichen den in der Praxis verwendeten Reibpaarungen. Um Reibpaarungen auf ihre Langzeittauglichkeit zu prüfen, verwendet man als Prüföl ein bewährtes Öl mit bekannten Eigenschaften.

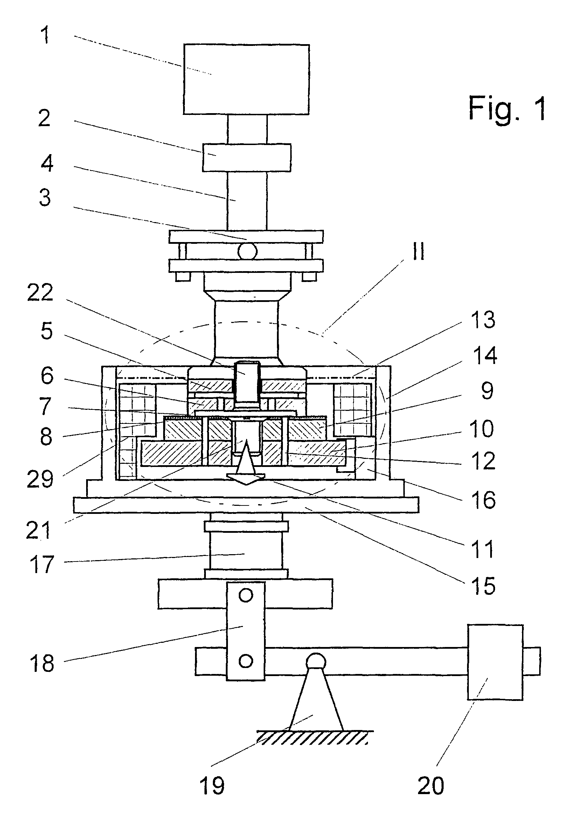

Es folgt der Abgleich der Meßwertaufnehmer. Um die Normalkraft zu messen, ist eine Kraftmeßeinrichtung 17 im Kraftfluß integriert, z. B. wie in Fig. 1 gezeigt zwischen dem Hebelmechanismus 18 und dem Prüftisch 15. Die Reibkraft wird mittels Meßstreifen an der Momentenabstützung 16 ermittelt. Das Antriebsmoment des Motors 1, der von einem externen Sollwertgeber auf eine vorgegebene Solldrehzahl geregelt wird, kann mittels der Drehmomentmeßnabe 2 erfaßt und alternativ, wenn auch mit einer größeren Ungenauigkeit, zur Ermittlung der Reibkraft verwendet werden. Danach wird die Prüflast aufgebracht, die sich aufgrund des Spitzenlagers 11 und er Ausgleichsverbindung 3 gleichmäßig auf die Ringfläche 7 verteilt.

Mit einem doppelwandigen, temperierbaren Gefäß als Prüfbehälter 14 wird die Prüftemperatur eingeregelt, wozu ein nicht dargestellter Temperaturfühler im Prüfbehälter 14 dient. Ist die Prüftemperatur erreicht, kann der Einlaufvorgang zur Formierung der Reibflächen an der Ringfläche 7 und am Reibbelag 8 gestartet werden. Daran schließt sich der eigentliche Prüfzyklus an, wobei eine Meßreihe mit mehreren Gleitgeschwindigkeiten durchgeführt wird, die durch die Solldrehzahl des Motors 1 vorgegeben werden. Der Reibwert wird rechnerisch aus dem Verhältnis der Reibkraft zur Normalkraft ermittelt.

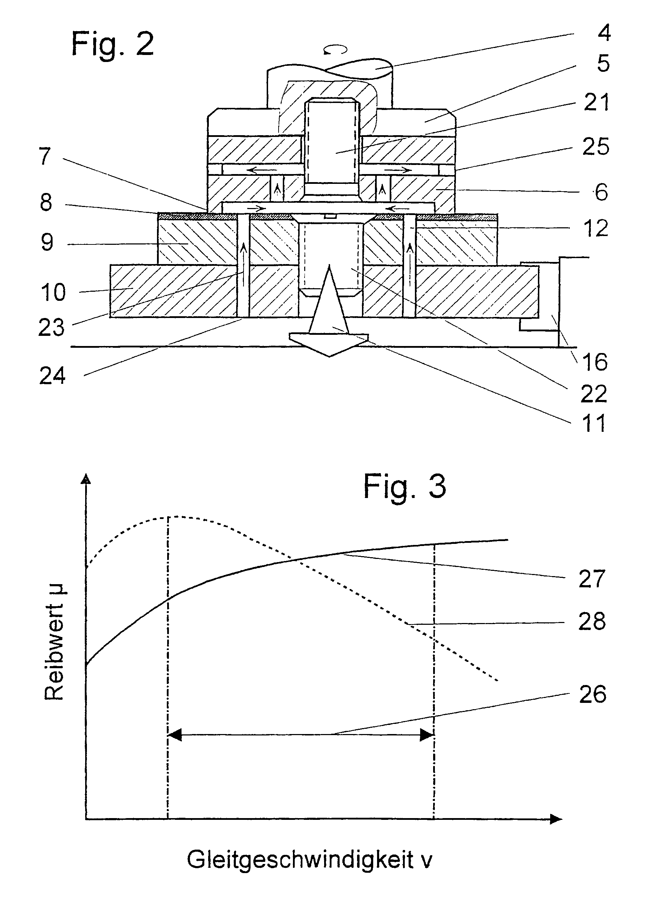

Fig. 3 zeigt ein Schaubild mit zwei Reibwertcharakteristiken 27 und 28. Verläuft die Reibwertcharakteristik 27 in einem Betriebsbereich 26 progressiv, ist das Schmiermittel für den Einsatz in schlupfgeregelten Kupplungen geeignet. Fällt jedoch die Reibwertcharakteristik 28 im Betriebsbereich 26 degressiv ab, ist im Fahrzeugeinsatz mit selbsterregten Reibschwingungen zu rechnen, so daß das Schmiermittel für diesen Einsatzfall nicht geeignet ist.

Um zu vermeiden, daß sich das Prüföl unter der Ringscheibe 6 überhitzt, sondern gleichmäßig temperiert bleibt, sind im Probenhalter 10 und im Grundkörper 9 sowie in der Ringscheibe 6 und der Stützscheibe 5 Ölkanäle 12 vorgesehen, durch die das Prüföl in Richtung der Pfeile 23 von einem Öleintritt 24 zu einem Ölaustritt 25 befördert wird. Die Förderwirkung wird durch Zentrifugalkraft in den im wesentlichen radial verlaufenden Teilstücken der rotierenden Ölkanäle 12 erzeugt.

Aufgrund des einfachen Aufbaus und der geringen Prüfölmenge, die für eine Meßwertreihe erforderlich ist, kann der Aufwand für die Messungen über einen Lebensdauerzyklus in vertretbaren Grenzen gehalten werden. Die geringe Prüfölentnahme erlaubt, daß Dauerversuche ohne Beeinträchtigung fortgeführt werden können, so daß man aussagekräftige Meßergebnisse für den gesamten Lebensdauerzyklus erhält.

### Bezugszeichen

- 1: Motor
- 2: Drehmomentmeßnabe
- 3: Ausgleichsverbindung
- 4: Antriebswelle
- 5: Stützscheibe
- 6: Ringscheibe
- 7: Ringfläche
- 8: Reibbelag
- 9: Grundkörper
- 10: Probenhalter
- 11: Spitzenlager
- 12: Ölkanäle
- 13: Ölspiegel
- 14: Püfbehälter
- 15: Prüftisch
- 16: Momentenabstützung
- 17: Kraftmeßeinrichtung
- 18: Hebelmechanismus
- 19: Fundament
- 20: Lastaufgabesystem
- 21: Schraube
- 22: Schraube
- 23: Pfeile
- 24: Öleintritt
- 25: Ölaustritt
- 26: Betriebsbereich
- 27: Reibwertcharakteristik
- 28: Reibwertcharakteristik
- 29: Verdrängerkörper

## Patentansprüche

1. Verfahren zur Ermittlung der Funktionstauglichkeit von Schmierölen für schlupfgeregelte Kupplungen mit den folgenden Schritten:
- zwei Reibpartner (6, 8) werden aufgespannt und in einen mit Prüföl gefüllten Prüfbehälter (14) gegeben,
- Meßwertaufnehmer für eine Normalkraft (17) und eine Reibkraft werden abgeglichen,
- ein Reibpartner (6, 7) wird angetrieben, während der andere Reibpartner (8) stillsteht,
- als Prüflast wird eine Normalkraft aufgebracht,
- nachdem eine vorgegebene Prüftemperatur erreicht ist, wird ein Einlaufvorgang zur Formierung der Prüfflächen der Reibpartner (6, 8) gestartet,
- verschiedene Geschwindigkeitsstufen werden durch entsprechende Antriebsdrehzahlen eingestellt und solange gehalten, bis sich ein stabiler Zustand einstellt,
- anschließend werden die Reibkraft und die Normalkraft ermittelt und daraus der Reibwert errechnet,
**dadurch gekennzeichnet, daß** zur Durchführung des Verfahrens eine Vorrichtung verwendet wird, bei der im Prüfbehälter (14) ein Probenhalter (10) drehfest auf einem Spitzen- (11) oder Kugellager schwenkbar gelagert ist, der einen Grundkörper (9) mit einem ersten Reibpartner (8) trägt, an dem ein zweiter Reibpartner in Form einer Ringscheibe (6) mit einer definierten Ringfläche (7) anliegt, wobei der zweite Reibpartner (6) von einem Motor (1) angetrieben wird und ein Lastaufgabesystem (20) den Prüfbehälter (14) mit dem ersten Reibpartner (8) gegen den zweiten Reibpartner (6, 7) drückt.

2. Verfahren nach Anspruch 1, **dadurch gekennzeichnet, daß** die Prüfölmenge auf eine Menge unter 200 ml begrenzt ist.

3. Verfahren nach Anspruch 1 oder 2, **dadurch gekennzeichnet, daß** im Prüfbehälter (14) Verdrängerkörper (29) angeordnet sind.

4. Vorrichtung zur Durchführung des Verfahrens nach einem der vorangehenden Ansprüche, **dadurch gekennzeichnet,**
- **daß** in dem drehfest angeordneten Prüfbehälter ein Probenhalter (10) drehfest auf einem Spitzen- (11) oder Kugellager schwenkbar gelagert ist, der einen Grundkörper (9) mit einem ersten Reibpartner (8) trägt, an dem ein zweiter Reibpartner in Form einer Ringscheibe (6) mit einer definierten Ringfläche (7) anliegt, wobei der zweite Reibpartner (6) von einem Motor (1) angetrieben wird und ein Lastaufgabesystem (20) den Prüfbehälter (14) mit dem ersten Reibpartner (8) gegen den zweiten Reibpartner (6, 7) drückt,
- **daß** ein Meßwertaufnehmer für die Normalkraft (17) und die Reibkraft (2, 16) und ein Meßwertaufnehmer für die Prüftemperatur vorgesehen sind und
- **daß** am Antriebsmotor (1) verschiedene Antriebsdrehzahlen einstellbar sind.

5. Vorrichtung nach Anspruch 4, **dadurch gekennzeichnet, daß** der Prüfbehälter (14) ein doppelwandiges, temperierbares Gefäß ist.

6. Vorrichtung nach Anspruch 4 oder 5, **dadurch gekennzeichnet, daß** zwischen dem Lastaufgabesystem (20) und dem Prüfbehälter (14)eine Kraftmeßeinrichtung (17) angeordnet ist.

7. Vorrichtung nach einem der vorangehenden Ansprüche 4 bis 6, **dadurch gekennzeichnet, daß** die Reibkraft an der Momentenabstützung (16) mittels Dehnungsmeßstreifen erfaßt wird.

8. Vorrichtung nach einem der vorangehenden Ansprüche 4 bis 7, **dadurch gekennzeichnet, daß** im Probenhalter (10) und im Grundkörper (9) sowie in der Ringscheibe (6) und in der Stützscheibe (5) Ölkanäle (12) vorgesehen sind, die einen Öleintritt (24) mit einem Ölaustritt (25) verbinden und durch die Prüföl infolge der Zentrifugalkraft in rotierenden, radial verlaufenden Abschnitten der Ölkanäle (12) gefördert wird.

## Claims

1. Process for determining the functioning capability of lubricating oils for slip-regulated clutches, the said process comprising the following steps:
- two friction partners (6, 8) are mounted and placed in a test receptacle (14) filled with test oil,
- transducers for a normal force (17) and a friction force are balanced,
- one friction partner (6, 7) is driven while the other friction partner (8) stands still,
- a normal force is applied as a test load,
- after a predetermined test temperatures has been reached, a running-in operation is started for the purpose of forming the test faces of the friction partners (6, 8),
- various speed stages are set by means of suitable rotational driving speeds and maintained until a stable condition sets in, and
- the friction force and normal force are then determined and the friction value calculated therefrom,
**characterised in that**, for performing the said method, use is made of a device in which a specimen-holder (10) is mounted in the test receptacle (14) so as to be pivotable on a conical (11) or ball bearing in a torsion-proof manner, which specimen-holder carries a basic body (9) with a first friction partner (8) against which a second friction partner in the form of an annular disc (6) rests via a defined annular face (7), the said second friction partner (6) being driven by a motor (1), and a load-assigning system (20) pressing the test receptacle (14) with the first friction partner (8) against the second friction partner (6, 7).

2. Process according to claim 1, **characterised in that** the quantity of test oil is limited to a quantity of less than 200 ml.

3. Process according to claim 1 or 2, **characterised in that** displacer bodies (29) are disposed in the test receptacle (14),

4. Device for performing the process according to one of the preceding claims, **characterised in that**
- a specimen-holder (10) is mounted in the test receptacle, which is disposed in a torsion-proof manner, so as to be pivotable on a conical (11) or ball bearing in a torsion-proof manner, which specimen-holder carries a basic body (9) with a first friction partner (8) against which a second friction partner in the form of an annular disc (6) rests via a defined annular face (7), the said second friction partner (6) being driven by a motor (1), and a load-assigning system (20) pressing the test receptacle (14) with the first friction partner (8) against the second friction partner (6, 7),
- that a transducer for the normal force (17) and the friction force (2, 16), and a transducer for the test temperature are provided, and
- that various rotational driving speeds can be set at the driving motor (1).

5. Device according to claim 4, **characterised in that** the test receptacle (14) is a double-wall, temperable vessel.

6. Device according to claim 4 or 5, **characterised in that** a force-measuring apparatus (17) is disposed between the load-assigning system (20) and the test receptacle (14).

7. Device according to one of the preceding claims 4 to 6, **characterised in that** the friction force at the moment-supporting device (16) is detected by means of strain gauges.

8. Device according to one of the preceding claims 4 to 7, **characterised in that** there are provided, in the specimen-holder (10) and basic body (9) and also in the annular disc (6) and supporting disc (5), oil ducts (12) which connect an oil entry (24) to an oil exit (25) and through which test oil is conveyed, as a result of centrifugal force, in rotating, radially extending sections of the said oil ducts (12).

## Revendications

1. Procédé de détermination de l'aptitude au fonctionnement d'huiles lubrifiantes pour embrayages à glissement réglé, comprenant les phases suivantes :
- deux partenaires de friction (6, 8) sont serrés l'un contre l'autre et placés dans un récipient d'essai (14) rempli de l'huile à essayer,
- des capteurs de valeurs mesurées sont étalonnés pour une force normale (17) et pour une force de frottement,
- un partenaire de friction (6, 7) est entraîné tandis que l'autre partenaire de friction (8) reste immobile,
- une force normale est appliquée en tant que charge d'essai,
- lorsqu'une température d'essai prédéterminée est atteinte, une opération de rodage est mise en marche pour la mise en forme des surfaces d'essai des partenaires de friction (6, 8).
- différents étages de vitesse sont établis par des vitesses de rotation d'entraînement correspondantes et maintenus jusqu'à ce qu'un état stable se soit établi,
- ensuite, la force de frottement et la force normale sont mesurées et le coefficient de frottement est calculé sur cette base,
**caractérisé en ce que**, pour la mise en oeuvre du procédé, on utilise un dispositif dans lequel, à l'intérieur du récipient d'essai (14), un support d'éprouvette (10) est monté solidairement en rotation et oscillant sur un palier à pointe (11) ou à billes, qui porte un corps de base (9) portant un premier partenaire de friction (8) contre lequel un deuxième partenaire de friction, de la forme d'un disque annulaire (6) est en appui par une surface annulaire définie (7), le deuxième partenaire de friction (6) étant entraîné par un moteur (1) et un système d'application de charge (20) pressant le récipient d'essai (14) qui contient le premier partenaire de friction (8) contre le deuxième partenaire de friction (6, 7).

2. Procédé selon la revendication 1, **caractérisé en ce que** la quantité d'huile d'essai est limitée à une quantité de moins de 200 ml.

3. Procédé selon la revendication 1 ou 2, **caractérisé en ce que** des corps de déplacement (29) sont disposés dans le récipient d'essai (14).

4. Dispositif pour la mise en oeuvre du procédé selon l'une quelconque des revendications précédentes, **caractérisé en ce que**
à l'intérieur du récipient d'essai, un support d'éprouvette (10) est monté solidairement en rotation et oscillant sur un palier à pointe (11) ou à billes, qui porte un corps de base (9) portant un premier partenaire de friction (8) contre lequel un deuxième partenaire de friction, de la forme d'un disque annulaire (6) est en appui par une surface annulaire définie (7), le deuxième partenaire de friction (6) étant entraîné par un moteur (1) et un système d'application de charge (20) pressant le récipient d'essai (14) qui contient le premier partenaire de friction (8) contre le deuxième partenaire de friction (6, 7).
il est prévu un détecteur de valeurs mesurées pour la force normale (17) et la force de frottement (2, 16) et un capteur de valeurs mesurées pour la température d'essai, et
différentes vitesses de rotation d'entraînement peuvent être établies sur le moteur d'entraînement (1).

5. Dispositif selon la revendication 4, **caractérisé en ce que** le récipient d'essai (14) est un récipient à double paroi pouvant être réglé en température.

6. Dispositif selon la revendication 4 ou 5, **caractérisé en ce qu'**un dispositif de mesure de la force (17) est disposé entre le système d'application de la charge (20) et le récipient d'essai (14).

7. Dispositif selon l'une quelconque des revendications précédentes, **caractérisé en ce que** la force de frottement est relevée sur le support de réaction de couple (16) au moyen de jauges de contraintes.

8. Dispositif selon l'une quelconque des revendications précédentes, **caractérisé en ce que**, dans le support d'éprouvette (10) et dans le corps de base (9), ainsi que dans le disque annulaire (6) et dans le disque d'appui (5), sont prévus des canaux d'huile (12) qui relient une entrée d'huile (24) à une sortie d'huile (25) et à travers lesquels de l'huile d'essai est refoulée sous l'effet de la force centrifuge dans des segments tournants d'orientation radiale des canaux d'huile (12).
